(19) Europäisches Patentamt / European Patent Office / Office européen des brevets

(11) **EP 3 177 727 B1**

(12) **EUROPEAN PATENT SPECIFICATION**

(45) Date of publication and mention
of the grant of the patent:
**12.12.2018 Bulletin 2018/50**

(21) Application number: **15733667.8**

(22) Date of filing: **23.06.2015**

(51) Int Cl.:
*C12P 7/06* *(2006.01)*     *C02F 3/34* *(2006.01)*
*C12F 3/10* *(2006.01)*     *C12P 7/14* *(2006.01)*
*A23K 10/38* *(2016.01)*     *C12P 3/00* *(2006.01)*
*C12P 7/10* *(2006.01)*     *C12P 7/40* *(2006.01)*

(86) International application number:
**PCT/EP2015/064101**

(87) International publication number:
**WO 2016/020101 (11.02.2016 Gazette 2016/06)**

(54) **DEWATERING METHODS IN FERMENTATION PROCESSES**

VERFAHREN ZUR ENTWÄSSERUNG IN FERMENTATIONSPROZESSEN

PROCÉDÉS DE DÉSHYDRATATION UTILISABLES DANS DES PROCESSUS DE FERMENTATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.08.2014 EP 14179848**
            **05.08.2014 US 201462033338 P**

(43) Date of publication of application:
**14.06.2017 Bulletin 2017/24**

(73) Proprietors:
• **Direvo Industrial Biotechnology GmbH**
**50829 Köln (DE)**
• **BASF Enzymes LLC**
**San Diego, CA 92121 (US)**

(72) Inventors:
• **KRÄMER, Marco**
**50829 Cologne (DE)**
• **SVETLITCHNYI, Vitaly**
**50829 Cologne (DE)**

(74) Representative: **Roth, Andy Stefan**
**Dr. Roth Patentanwaltskanzlei**
**Kaistrasse 5**
**40221 Düsseldorf (DE)**

(56) References cited:
**WO-A1-2007/056321**     **WO-A1-2012/084225**
**WO-A2-2011/126897**

• **JINGBO LI ET AL: "Monosaccharides and
Ethanol Production from Superfine Ground
Sugarcane Bagasse Using Enzyme Cocktail",
BIORESOURCES, NORTH CAROLINA STATE
UNIVERSITY * COLLEGE OF NATURAL
RESOURCES, US, vol. 9, no. 2, 21 March 2014
(2014-03-21), pages 2529-2540, XP002743195,
ISSN: 1930-2126**
• **JINGBO LI ET AL: "Synergism of cellulase,
xylanase, and pectinase on hydrolyzing
sugarcane bagasse resulting from different
pretreatment technologies", BIORESOURCE
TECHNOLOGY, ELSEVIER BV, GB, vol. 155, 1
March 2014 (2014-03-01), pages 258-265,
XP002743230, ISSN: 0960-8524, DOI:
10.1016/J.BIORTECH.2013.12.113 [retrieved on
2014-01-04]**

**Description**

**FIELD OF THE INVENTION**

The present disclosure relates to an improved process for dewatering the whole stillage in fermentation processes.

**BACKGROUND OF THE INVENTION**

Fermentation products, such as ethanol, are produced by first degrading starch- containing material into fermentable sugars by liquefaction and saccharification and then converting the sugars directly or indirectly into the desired fermentation product using a fermenting organism. Liquid fermentation products such as ethanol are recovered from the fermented mash (often referred to as "beer" or "beer mash"), e.g., by distillation, which separate the desired fermentation product from other liquids and/or solids. The remaining faction, referred to as "whole stillage", is dewatered and separated into a solid and a liquid phase, e.g., by centrifugation. The solid phase is referred to as "wet cake" (or "wet grains" or "WDG") and the liquid phase (supernatant) is referred to as "thin stillage". Dewatered wet cake is dried to provide "Distillers Dried Grains" (DDG) used as nutrient in animal feed. Thin stillage is typically evaporated to provide condensate and syrup (or "thick stillage") or may alternatively be recycled directly to the slurry tank as "backset". Condensate may either be forwarded to a methanator before being discharged or may be recycled to the slurry tank. The syrup consisting mainly of limit dextrins and non-fermentable sugars may be blended into DDG or added to the wet cake before drying to produce DDGS (Distillers Dried Grain with Solubles).

Ethanol plants have struggled to maintain profitability, which is highly variable depending upon corn price, demand and price of DDGS, tax credits, gasoline consumption, ethanol exports, and changes to the Renewable Fuels Standard (RFS) mandates. New technologies for energy savings, higher yield of ethanol and higher value for co-products as well as various oil separation technologies contribute to the profitability of producing ethanol.

WO2012/084225 and WO2007/056321 describe the treatment of the fermented mash (beer) after the fermentation with enzymes or enzyme compositions for dewatering the whole stillage.

WO2011/126897 discloses the use of an enzyme composition comprising a hemicellulase, an endoglucanase and GH61 polypeptide in a fermentation process for dewatering whole stillage.

US patent application no. 2005/0079270 A1 discloses a method of dewatering corn stillage solids comprising adding to the solids an anionic copolymer comprising acrylic acid sodium salt, methacrylic acid sodium salt or 2-acrylamido-2-methyl-1-propanesulfonic acid sodium salt to form a mixture of water and coagulated and flocculated solids; and separating the water from the coagulated and flocculated solids using a dewatering device.

Dewatering of whole stillage is energy demanding and may consume up to one-third of the energy requirement of a plant producing ethanol or a similar fermentation product. Thus, there is a need for improving processes involved in dewatering of whole stillage.

**SUMMARY OF THE DISCLOSURE**

The present disclosure relates to a method of dewatering stillage obtained from a process for producing a fermentation product.

The present disclosure relates to methods for dewatering a whole stillage in a fermentation process, comprising:

(a) saccharifying a cellulosic material with an enzyme composition;
(b) fermenting the saccharified cellulosic material with a fermenting organism to produce a fermentation product, wherein the fermentation medium comprises a xylanase and a pectinase;
(c) distilling the fermentation product to form the whole stillage; and
(d) separating the whole stillage into thin stillage and wet cake, whereby more liquid phase is transferred to the thin stillage,

wherein the cellulosic material is corn and the fermentation product is ethanol.

In a process for increasing the dewatering of the whole stillage, the liquefied whole grain mash can be thinned by treatment with an efficient amount of enzyme activity of a xylanase in combination with a pectinase.

The process for dewatering a whole stillage in a fermentation process, can sequentially comprise the following steps: a) milling whole grain; b) liquefying the gelatinised milled whole grain, in the presence of an alpha-amylase; c) saccharifying the liquefied material in the presence of a glucoamylase; d) fermentation with a microorganism; e) distillation of fermented and saccharified material, providing an ethanol fraction, wherein the liquefied mash is subjected to an effective amount enzyme activity of a xylanase and a pectinase during the fermentation.

**BRIEF DESCRIPTION OF THE DRAWING**

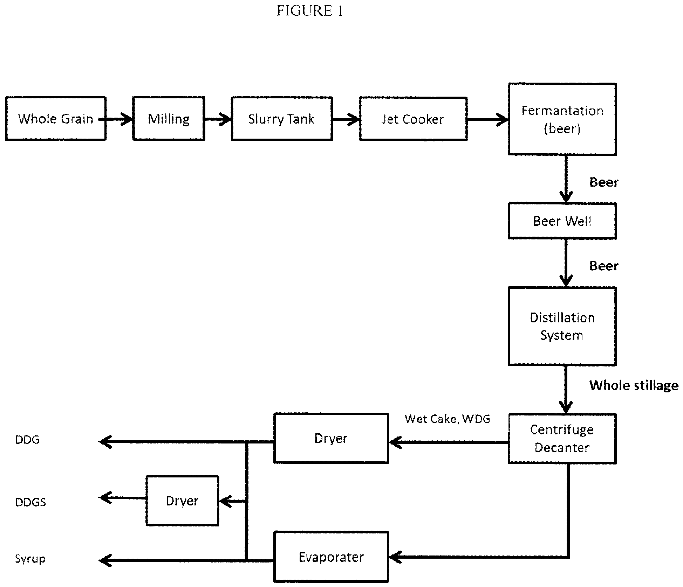

Figure 1 schematically shows an ethanol production process.

**DESCRIPTION OF THE INVENTION**

The object of the present invention is to provide a method of dewatering whole stillage. The addition of a xylanase in combination with a pectinase to the fermentation medium results in a wet cake with a higher dry mass. The advantage here is less energy consumption while drying.

The present disclosure relates to methods for dewatering a whole stillage in a fermentation process, comprising:

(a) saccharifying a cellulosic material with an enzyme composition;
(b) fermenting the saccharified cellulosic material with a fermenting organism to produce a fermentation product, wherein the fermentation medium comprises a xylanase and a pectinase;
(c) distilling the fermentation product to form the whole stillage; and
(d) separating the whole stillage into thin stillage and wet cake, whereby more liquid phase is transferred to the thin stillage,

wherein the cellulosic material is corn and the fermentation product is ethanol.

Stillage or Whole stillage is the product which remains after the mash has been converted to sugar, fermented and distilled into ethanol. Stillage can be separated into two fractions, such as, by centrifugation or screening: (1) wet cake (solid phase) and (2) the thin stillage (supernatant). The solid fraction or distillers' wet grain (DWG) can be pressed to remove excess moisture and then dried to produce distillers' dried grains (DDG). After ethanol has been removed from the liquid fraction, the remaining liquid can be evaporated to concentrate the soluble material into condensed distillers' solubles (DS) or dried and ground to create distillers' dried solubles (DDS). DDS is often mixed with DDG to form distillers' dried grain with solubles (DDGS). DDG, DDGS, and DWG are collectively referred to as distillers' grain(s).

The enzymes xylanase and pectinase are added during the fermentation. The enzymes were capable of degrading components in the fermentation medium.

The phrase "fermentation media" or "fermentation medium" refers to the environment in which fermentation is carried out and comprises the fermentation substrate, that is, the carbohydrate source that is metabolized by the fermenting organism(s).

The fermentation medium may comprise other nutrients and growth stimulator(s) for the fermenting organism(s). Nutrient and growth stimulators are widely used in the art of fermentation and include nitrogen sources, such as ammonia; vitamins and minerals, or combinations thereof. Subsequent to fermentation, the fermentation product may be separated from the fermentation medium. The fermentation medium may be distilled to extract the desired fermentation product or the desired fermentation product may be extracted from the fermentation medium by micro or membrane filtration techniques. Alternatively, the fermentation product may be recovered by stripping. Methods for recovery are well known in the art.

The feedstock for producing ethanol is corn.

Production of a fermentation product is typically divided into the following main process stages:

a) Reducing the particle size of starch-containing material, e.g., by dry or wet milling;
b) Cooking the starch-containing material in aqueous slurry to gelatinize the starch,
c) Liquefying the gelatinized starch-containing material in order to break down the starch (by hydrolysis) into maltodextrins (dextrins);
d) Saccharifying the maltodextrins (dextrins) to produce low molecular sugars (e.g., DP1-2) that can be metabolized by a fermenting organism;
e) Fermenting the saccharified material using a suitable fermenting organism directly or indirectly converting low molecular sugars into the desired fermentation product;
f) Recovering the fermentation product, e.g., by distillation in order to separate the fermentation product from the fermentation mash.

As also explained in the "Background"-section above whole stillage is a by-product consisting of liquids and solids remaining after recovery (e.g. by distillation) of a desired fermentation product from fermented mash (beer mash). According to the invention the fermentation product.

The whole stillage contemplated according to the present disclosure is the side-product resulting from a fermentation product production process including above mentioned steps a) to c).

Dewatering of whole stillage, in order to remove a significant portion of the liquid/water, may be done using any suitable separation technique, including centrifugation, pressing and filtration. The whole stillage may be heated to a temperature of about 20-60° C. The pH is in the range from 3-7, preferably pH 3-6.

In a preferred embodiment the dewatering is carried out by centrifugation. Preferred centrifuges in industry today are decanter type centrifuges, preferably high speed decanter type centrifuges. An example of a suitable centrifuge is the NX 400 steep cone series from Alfa Laval which is a high-performance decanter.

In another preferred embodiment the separation is carried out using other conventional separation equipment such as a plate/frame filter presses, belt filter presses, screw presses, gravity thickeners and deckers, or similar equipment.

After the wet cake, containing about 30-35 wt-% dry solids, has been dewatered it may be dried in a drum dryer, spray dryer, ring drier, fluid bed drier or the like in order to produce "Distillers Dried Grains" (DDG). DDG is a valuable feed ingredient for livestock, poultry and fish. It is preferred to provide DDG with a content of less than about 10-12 wt.-% moisture to avoid mold and microbial breakdown and increase the shelf life. Further, high moisture content also makes it more expensive to transport DDG. The wet cake is preferably dried under conditions that do not denature proteins in the wet cake. The wet cake may be blended with syrup separated from the thin stillage fraction and dried into DDG with Solubles (DDGS).

Corn is the feedstock and the fermentation product is ethanol.

As mentioned above beer (or fermented mash) comprises the fermentation product ethanol.

The fermenting organism may be a fungal organism, such as yeast, or bacteria. Suitable bacteria may e.g. be *Zymomonas* species, such as *Zymomonas mobilis* and *E. coli*. Examples of filamentous fungi include strains of *Penicillium* species. Preferred organisms for ethanol production are yeasts, such as e.g. *Pichia* or *Saccharomyces*. Preferred yeasts according to the disclosure are *Saccharomyces* species, in particular *Saccharomyces cerevisiae* or baker's yeast.

Further, by adding the enzymes to the fermentation medium before the distillation step is an advantage since the enzymes in the enzyme compositions are inactivated during the distillation.

Processes for producing ethanol from corn typically begins with grinding the corn in a dry-grind or wet-milling process. Wet-milling processes involve fractionating the corn into different components where only the starch fraction enters into the fermentation process. Dry-grind processes involve grinding the corn kernels into meal and mixing the meal with water and enzymes. Generally two different kinds of dry-grind processes are used. The most commonly used process, often referred to as a "conventional process," includes grinding the starch-containing material and then liquefying gelatinized starch at a high temperature using typically a bacterial alpha-amylase, followed by simultaneous saccharification and fermentation (SSF) carried out in the presence of a glucoamylase and a fermentation organism. Another well-known process, often referred to as a "raw starch hydrolysis" process (RSH process), includes grinding the starch-containing material and then simultaneously saccharifying and fermenting granular starch below the initial gelatinization temperature typically in the presence of an acid fungal alpha-amylase and a glucoamylase.

In a process for producing ethanol from corn, following SSF or the RSH process the ethanol is distilled from the whole mash after fermentation. The resulting ethanol-free slurry, usually referred to as whole stillage, is separated into solid and liquid fractions (i.e., wet cake and thin stillage containing about 35 and 7% solids, respectively). The thin stillage is often condensed by evaporation into a thick stillage or syrup and recombined with the wet cake and further dried into distillers' dried grains with solubles distillers' dried grain with solubles (DDGS) for use in animal feed. In the method as claimed, SSF and RSH are not used.

The xylanase may preferably be of microbial origin, such as of fungal origin (e.g., Aspergillus, Fusarium, Humicola, Meripilus, Trichoderma) or from a bacterium (e.g., Bacillus). In a preferred embodiment the xylanase is derived from a filamentous fungus, preferably derived from a strain of Aspergillus, such as Aspergillus aculeatus; or a strain of Humicola, preferably Humicola lanuginosa. Examples of xylanases useful in the methods of the present invention include, but are not limited to, Aspergillus aculeatus xylanase (GeneSeqP:AAR63790; WO 94/21785), Aspergillus fumigatus xylanases (WO 2006/078256), and Thielavia terrestris NRRL 8126 xylanases (WO 2009/079210). The xylanase may preferably be an endo-1,4-beta-xylanase, more preferably an endo-1,4-beta-xylanase of GH 10 or GH 1 1. Examples of commercial xylanases include SHEARZYME(TM), BIOFEED WHEAT(TM), HTec and HTec2 from Novozymes A/S, Denmark.

Examples of beta-xylosidases useful in the methods of the present invention include, but are not limited to, Trichoderma reesei beta-xylosidase (UniProtKB/TrEMBL accession number Q92458), Talaromyces emersonii (Swiss-Prot accession number Q8X212), and Neurospora crassa (SwissProt accession number Q7SOW4).

Examples of suitable bacterial xylanases include include xylanases derived from a strain of Bacillus, such as Bacillus subtilis, such as the one disclosed in US patent no. 5,306,633.

Contemplated commercially available xylanases include SHEARZYM E (TM), BIOFEED WHEAT(TM), (from Novozymes AJS), Econase CE™ (from AB Enzymes), Depol 676™ (from Biocatalysts Ltd.) and SPEZYME(TM) CP (from Genencor Int.).).

Xylanase may be added in an amount effective in the range from $0.16 \times 10^6$ - $460 \times 10^6$ Units per ton beer mash or fermentation medium.

Determination of Xylanase Activity (FXU)

The endoxylanase activity is determined by an assay, in which the xylanase sample is incubated with a remazol-xylan substrate (4-O-methyl-D-glucurono-D-xylan dyed with Remazol Brilliant Blue R, Fluka), pH 6.0. The incubation is performed at 50°C. for 30 min. The background of non-degraded dyed substrate is precipitated by ethanol. The remaining blue colour in the supernatant is determined spectrophotometrically at 585 nm and is proportional to the endoxylanase activity. The endoxylanase activity of the sample is determined relatively to an enzyme standard.

The pectinase used in the methods according to the present disclosure may be any pectinase, in particular of microbial origin, in particular of bacterial origin, such as a pectinase derived from a species within the genera Bacillus, Clostridium, Pseudomonas, Xanthomonas and Erwinia, or of fungal origin, such as a pectinase derived from a species within the genera Trichoderma or Aspergillus, in particular from a strain within the species A. niger and A. aculeatus. Contemplated commercially available pectinases include Pectinex Ultra-SPL™ (from Novozymes), Pectinex Ultra Color (from Novozymes), Rohapect Classic (from AB Enzymes), Rohapect 10L (from AB Enzymes). Pectinase may be added in an amount effective in the range from $1.4 \times 10^9$ - $23500 \times 10^9$ Units per ton fermentation medium.

Determination of Pectintranseliminase Unit (PECTU)

The method is based on the enzyme's degradation of a pectin solution by a transeliminase reaction, the double bonds formed result in an increase in the absorption at 238 nm which is followed by a spectrophotometer.

Reaction conditions

Temperature: 30°C. $\pm$ 0.5° C.
pH: 3.50 $\pm$ 0.02
Substrate: 0.24% Pectin (Obipektin, Brown Ribbon Pure, Art. no. 1.1B00.A. Lot no. 0304)
Enzyme concentration: 1.9-2.3 PECTU/mL
Reaction time: 6 minutes
Measuring time: 5 minutes
Wavelength: 238 nm

The activity is determined relative to a PECTU standard. The result is given in the same units as for the standard, which is designated: PECTU-Pectintranseliminase Unit.

The term "alpha-amylase" means an alpha-1,4- glucan-4-glucanohydrolase (E.C. 3.2.1.1) that catalyzes the hydrolysis of starch and other linear and branched 1,4-glucosidic oligo- and polysaccharides.

In an embodiment, the xylanase is added in an amount of 1-30, e.g., 5-30 7-25, 10-20, 10-17, or 12-15 micrograms/g dry solids.

In an embodiment, the pectinase is added in an amount of 0.01-1.0, e.g., 0.015- 0.08, 0.015-0.06, 0.015-0.04, or 0.02-0.03 FXU/g dry solids.

The saccharification and fermentation steps are carried out sequentially. The xylanase and the pectinase are added during the fermentation.

As mentioned above, the fermenting organism is preferably yeast, e.g., a strain of *Saccharomyces cerevisiae* or *Saccharomyces diastaticus.* In an avantegeous embodiment a yeast strain of *Saccharomyces diastaticus* is used (SIHA Amyloferm®, E. Begerow GmbH&Co, Langenlonsheim, Germany) since their exo-amylase activity can split liquid starch and also dextrin, maltose and melibiose.

In the liquefaction step the gelatinized starch (downstream mash) is broken down (hydrolyzed) into maltodextrins (dextrins). To achieve starch hydrolysis a suitable enzyme, preferably an alpha-amylase, is added. Liquefaction may be carried out as a three-step hot slurry process. The slurry is heated to between 60-95°C, preferably 80-85°C, and an alpha-amylase may be added to initiate liquefaction (thinning). Then the slurry may be jet-cooked at a temperature between 95-140°C, preferably 105-125°C, for about 1-15 minutes, preferably for about 3-10 minutes, especially around about 5 minutes. The slurry is cooled to 60-95°C and more alpha-amylase may be added to complete the hydrolysis (secondary liquefaction). The liquefaction process is usually carried out at a pH of 4.0 to 6.5, in particular at a pH of 4.5 to 6.

The saccharification step and the fermentation step are performed as separate process steps. The saccharification is carried out in the presence of a saccharifying enzyme, e. g. a glucoamylase, a beta-amylase or maltogenic amylase. Optionally a phytase and/or a protease is added.

Saccharification may be carried out using conditions well known in the art with a saccharifying enzyme, e.g., beta-amylase, glucoamylase or maltogenic amylase, and optionally a debranching enzyme, such as an isoamylase or a pullulanase. For instance, a full saccharification process may last up to from about 24 to about 72 hours.

Saccharification is typically carried out at a temperature from 20-75°C, preferably from 40-70°C, typically around 60°C, and at a pH between 4 and 5, normally at about pH 4.5.

During the fermentation, the fermentation medium is subjected to an enzyme composition according to the present disclosure. The enzyme composition comprises a xylanase and a pectinase.

The process of the present disclosure may further comprise, prior to liquefying the starch-containing material the steps of:

- reducing the particle size of the starch-containing material, preferably by milling; and
- forming a slurry comprising the starch-containing material and water.

The aqueous slurry may contain from 10-55 w/w % dry solids (DS), preferably 25-45 w/w % dry solids (DS), more preferably 30-40 w/w % dry solids (DS) of the starch-containing material. The slurry is heated to above the gelatinization temperature and an alpha-amylase, preferably a bacterial and/or acid fungal alpha-amylase, may be added to initiate liquefaction (thinning). The slurry may be jet-cooked to further gelatinize the slurry before being subjected to an alpha-amylase in step (a).

The starch containing material is corn which can be milled and the methods comprise a step of milling the corn before step (a). In other words, the disclosure also encompasses methods, wherein the starch containing material is obtainable by a process comprising milling of corn, preferably dry milling, e. g. by hammer or roller mils.

Grinding is also understood as milling, as is any process suitable for opening the individual grains and exposing the endosperm for further processing. Two processes of milling are normally used in alcohol production: wet and dry milling. The term "dry milling" denotes milling of the whole grain. In dry milling the whole kernel is milled and used in the remaining part of the process Mash formation. The mash may be provided by forming a slurry comprising the milled starch containing material and brewing water. The brewing water may be heated to a suitable temperature prior to being combined with the milled starch containing material in order to achieve a mash temperature of 45 to 70°C, preferably of 53 to 66°C, more preferably of 55 to 60°C. The mash is typically formed in a tank known as the slurry tank.

Subsequent to fermentation the fermentation product ethanol may be separated from the fermentation medium. The slurry may be distilled to extract the ethanol from the fermentation medium by micro or membrane filtration techniques. Alternatively the fermentation product may be recovered by stripping. Methods for recovering fermentation products are well known in the art. Typically, ethanol with a purity of up to, e.g., about 96 vol. % ethanol is obtained.

Following the completion of the fermentation process, the material remaining is considered the whole stillage. As used herein, the term "whole stillage" includes the material that remains at the end of the fermentation process after recovery of ethanol. The fermentation product can optionally be recovered by any method known in the art. The whole stillage is separated or partitioned into a solid and liquid phase by one or more methods for separating the thin stillage from the wet cake. Such methods include, for example, centrifugation and decanting. The fermentation product can be recovered before the whole stillage is separated into a solid and liquid phase.

Thus, the methods of the disclosure comprise distillation to obtain the fermentation product ethanol. The fermentation and the distillation are carried out sequentially optionally followed by one or more process steps for further refinement of the fermentation product.

The aqueous by-product (whole stillage) from the distillation process is separated into two fractions, e.g., by centrifugation: wet grain (solid phase), and thin stillage (supernatant). The methods of the disclosure further comprise separation of the whole stillage produced by distillation into wet grain and thin stillage; and may comprise recycling thin stillage to the starch containing material prior to liquefaction. The thin stillage may be recycled to the milled whole grain slurry. The wet grain fraction may be dried, typically in a drum dryer. The dried product is referred to as distillers dried grains, and can be used as mentioned above as high quality animal feed. The thin stillage fraction may be evaporated providing two fractions (see Fig. 1 (i) a condensate fraction of 4-6% DS (mainly of starch, proteins, oil and cell wall components), and (ii) a syrup fraction, mainly consisting of limit dextrins and non-fermentable sugars, which may be introduced into a dryer together with the wet grains (from the whole stillage separation step) to provide a product referred to as distillers dried grain with solubles, which also can be used as animal feed. Thin stillage is the term used for the supernatant of the centrifugation of the whole stillage. Typically, the thin stillage contains 4-6% DS (mainly starch and proteins) and has a temperature of about 60-90°C. In another embodiment, the thin stillage is not recycled, but the condensate stream of evaporated thin stillage is recycled to the slurry containing the milled whole grain to be jet cooked.

Further details on how to carry out liquefaction, saccharification, fermentation, distillation, and recovering of ethanol are well known to the skilled person.

Methods for dewatering stillage from a fermentation product are known in the art. These methods include decanting or otherwise separating the whole stillage into wet cake and thin stillage. See, e.g., U.S. Patent Nos. 6,433,146, 7,601,858, and 7,608,729, and U.S. Application Publication No. 2010/0058649.

The fermentation product ethanol can be optionally recovered from the fermentation medium using any method known in the art including, but not limited to, chromatography, electrophoretic procedures, differential solubility, distillation, or extraction. For example, alcohol is separated from the fermented cellulosic material and purified by conventional methods of distillation as mentioned above. Ethanol with a purity of up to about 96 vol.% can be obtained, which can be used as, for example, fuel ethanol, drinking ethanol, i.e., potable neutral spirits, or industrial ethanol.

The present invention is further described by the following examples.

EXAMPLES

**De-watering of whole stillage**

The specification, a better dewatering capability of the whole stillage results in a wet cake with a higher dry mass. The advantage here is less energy consumption while drying.

For that example the dewatering capability of the whole stillage during the centrifugation was tested and determined.

Four different setups were tested.

In the first fermentor (Fermentor#1) the fermentation cultivation was not treated with enzymes (0 g/t xylanase; 0 g/t pectinase), in the second, third and forth fermentor (Fermentor#2 to #4) the fermentation cultivation was treated with the enzyme composition comprising a xylanase and a pectinase from the beginning of the fermentation in different concentrations (from 25 g/t to 200 g/t xylanase and from 25 g/t to 200 g/t pectinase).

The trials were performed in 2 L fermentation scale. After distillation the samples of the fermenters with the whole stillage were taken and the whole stillage was centrifuged to obtain thin stillage and the wet cake. De-watering [%] was calculated from the mass of thin stillage [mg] divided by the mass of whole stillage [mg] multiplied by 100.

In one embodiment, the process of the production of ethanol from corn was performed as follows:

**A) Process for producing fermentation products**

a) Reducing the particle size of the starch-containing material by milling

- corn (Company Pannonia, Hungary) was milled to < 2 mm particle size (coffee mill, company Brunn)

b) Forming a slurry comprising the starch-containing material and water

- 1,5 kg milled corn was added to 4,96 L ml warm tap water (water hardness 3,57 mmol/L) at 35°C to obtain a 25 % solid solution with a final volume of 6 L in a Biostat C fermentor (company Sartorius) leading to a pH of about 5,6.

c) Liquefying of the starch-containing material

- temperature was increased to 90°C

- 1 ml $\alpha$-amylase "$\alpha$-amylase VF-Kartoffel" (Schliessmann, Nr. 5049) was diluted in 10 ml tap water and then the diluted amylase was added to the slurry

- temperature was increased to 90°C

- the fermentor was incubated for 90 min at 90°C and 450 rpm

- the slurry was cooled to 30°C, pH was adjust to ~4 with 30% $H_2SO_4$

d) Saccharifying of the liquefied material obtained

- 1,5 ml glucoamylase "Amylase GA 500" (Schliessmann, Nr. 5042) was diluted in 10 ml sterile tap water and then the diluted glucoamylase was added to the slurry, which is the saccharified liquefied material.

e) Fermentation

- 1,8 g (NH4)2SO4 (i.e. 300 ppm ammonium sulphate) was added to the 6 L saccharified liquefied material.

- the saccharified liquefied material containing 300 ppm ammonium sulphate in the Biostat C fermentor was stirred with 800 rpm for 5 min to distribute everything evenly.

- the saccharified liquefied material containing 300 ppm ammonium sulphate (mash) was distributed in 1500 g single portions into four 2L Biostat B fermentors (company Sartorius) containing a horseshoe mixer.

- Enzyme stock preparation: 2,5 g of the pectinase (Pec3) with 90349 U/mL and 2,5 g of the xylanase (Xyl16) with 10027 U/mL were added into a 50 mL graduated cylinder and filled to 50 mL with tap water.

The enzyme stock was transferred into a 50 mL tube and then stored at 4°C until use within one hour.

- The following volumes of the enzyme stock preparation were added to the 2L Biostat B fermentor containing 1500 g of the saccharified liquefied material containing 300 ppm ammonium sulphate.

  Fermentor #1: 0 mL of the enzyme stock preparation leading to 0 g/t of pectinase and 0 g/t of xylanase

  Fermentor #2: 0,75 mL of the enzyme preparation leading to 25 g/t of pectinase and 25 g/t of xylanase

  Fermentor #3: 2,25 mL of the enzyme preparation leading to 75 g/t of pectinase and 75 g/t of xylanase

  Fermentor #4: 6,00 mL of the enzyme preparation leading to 200 g/t of pectinase and 200 g/t of xylanase

- Yeast propagation: 300 ml autoclaved YNB (yeast nitrogen base) medium plus glucose with 10 g/L glucose medium resulting in pH 5,7 in a 1L cultivation flasks, which had been inoculated with 2 ml yeast (Ethanol RED, company Fermentis) from a -80°C cryo stock containing 20% glycerol, were incubated for 23 hours (30°C, 150 rpm) leading to the yeast culture.

- Each of the four fermentors (Fermentor #1 to #4) was inoculated by 60 mL of the yeast culture.

- Cultivations of the fermentors were carried out at 30°C at 150 rpm, without pH control for 92,5 hours.

- 7-ml samples were taken twice the day by cut off pipette to monitor fermentation progress (ethanol concentration). The samples were transferred in 15 mL tubes and centrifuged at 4470 g for 10 minutes at 4°C and stored until further analysis at -20°C.

f) Ending the fermentation process and distillation of ethanol

- To end the fermentation process and to distillate ethanol the four fermentors were incubated at 80°C for 20 min and stirred at 150 rpm.

g) Taking samples for analysis of dewatering

- After the 20 min at 80°C and stirring at 150 rpm 3 x 40 to 45 ml samples were taken from each fermentor by a 50 mL pipette and filled into 50 mL tubes (samples of whole stillage).

**B) Enzyme product activity determination:**

DNSA solution: For the DNSA solution the following compounds were used:

- 5,00 g 3,5-Dinitrosalicylic acid (DNSA) was dissolved in 300 ml distilled $H_2O$.

- add 50 ml NaOH/KOH-solution (4M KOH + 4 M NaOH) drop per drop

- add 150 g K-Na-tartrate tetrahydrate

- cool solution to room temperature

- ad with destilled $H_2O$ to 500 ml final volume

- store in the darkness

*a) Pectinase*

Substrates: Polygalacturonic acid (Sigma 81325)

Substrates were dissolved in buffer to a concentration of 0,8% (w/v)

Buffer: 50 mM sodium acetate, pH 4,5

For the assay 96 well PCR microtiter plates (company Greiner) were used. The enzymes were diluted in buffer. 90 $\mu$l substrate and 10 $\mu$l enzyme solution were mixed. A blank was measured replacing enzyme solution with water. Incubation was carried out for 30 min at 37°C, followed by a 5 minute enzyme inactivation step at 99°C and followed by cooling for 10 min at 4°C. In a second 96 well PCR microtiter plates (company Greiner) 50 $\mu$l of the incubated substrate - enzyme mix was incubated with 50 $\mu$l of the DNSA solution at 98°C for 10 minutes and then cooled to 4°C and incubated for 5 minutes at 4°C.
100 $\mu$l of the reaction was transferred into a well of 96 well transparent, flat bottom micro titer plate and the adsorption was measured at 540 nm by a micro titer plate reader (Tecan M1000).

*b) Xylanase*

Substrates: Xylan from birchwood (Sigma X0502)

Substrate was dissolved in buffer to a concentration of 1,5% (w/v)

Buffer: 100 mM sodium acetate, pH 5,0 containing 20 mM $CaCl_2$ and 0,4g/L Tween20

For the assay 96 well PCR microtiter plate (company Greiner) were used. The enzymes were diluted in buffer. 90 $\mu$l substrate and 10 $\mu$l enzyme solution were mixed. A blank was measured replacing enzyme solution with water. Incubation was carried out for 20 min at 40°°C, followed by a 5 minute enzyme inactivation step at 99°C and followed by cooling for 5 min at 4°C. 45,5$\mu$l of the DNSA solution was added to the 96 well PCR microtiter plate by a multidrop (company Fisher-Scientific) and then the plate was incubated for 98°C for 10 minutes and cooled to 4°C and incubated for 5 minutes at 4°C.
100 $\mu$l of the reaction was transferred into well of a new 96 well transparent, flat bottom micro titer plate and then the adsorption was measured at 540 nm by a micro titer plate reader (Tecan M1000).
The activity is calculated as Units per $\mu$l or mg of enzyme product. 1 unit is defined as the amount of formed reducing ends in $\mu$mol per minute. The enzyme activities are shown in Table 1.

Table 1

| Type | Activity | Shortname |
|------|----------|-----------|
| pectinase | 90349 U/mL | *Pec3* |
| xylanase | 10027 U/mL | *Xyl16* |

**Example 1**

After the fermentation process and distillation of ethanol the following steps were made to analyze de-watering:

Determination of the mass of the whole stillage

1. About 40 ml of the whole stillage after distillation at 80°C were transferred into a 50 mL Falcon tube (FT1) and the mass was determined (mass FT1sample). The mass of the empty FT1 was previously determined (mass FT1empty). FT1 with the sample (FT1sample) was centrifuged at 800 g for 10 minutes.

Determination of the mass of the thin stillage

2. The supernatant was decanted into a new 50 mL Falcon tube (FT2) leading to the thin stillage and the mass was determined (mass FT2sample). The mass of FT2 was previously determined (mass FT2empty).

*Calculation of sample mass:*

$$\text{Mass of whole stillage [mg]} = (\text{mass FT1sample - mass FT1empty})$$

$$\text{Mass of thin stillage (supernatant) [mg]} = (\text{mass FT2sample - mass FT2empty})$$

*Calculation of dewatering*

$$\text{Dewatering [\%]} = (\text{Mass of thin stillage [mg] / Mass of whole stillage [mg]}) * 100$$

Table 2.

| added xylanase [g/t] | added pectinase [g/t] | Dewatering [%] | Related to control [0 g/t = 100%] |
|----------------------|-----------------------|----------------|-----------------------------------|
| 0 | 0 | 68,5 | 100,0% |
| 25 | 25 | 70,9 | 103,5% |
| 75 | 75 | 71,3 | 104,0% |
| 200 | 200 | 71,7 | 104,6% |

Table 2 shows the amount of thin stillage (supernatant) in % of whole stillage removed after centrifugation at 800 rpm for 10 minutes. Here it was shown that a treatment with the enzyme composition comprising a xylanase and a pectinase increases the dewatering capability by 3,5% using 25 ppm of each enzyme, 4,0% using 75 ppm of each enzyme and 4,6% using 200 ppm of each enzyme.

**Claims**

1. A method for dewatering a whole stillage in a fermentation process, comprising:

(a) saccharifying a cellulosic material with an enzyme composition;
(b) fermenting the saccharified cellulosic material with a fermenting organism to produce a fermentation product, wherein the fermentation medium comprises a xylanase and a pectinase;
(c) distilling the fermentation product to form the whole stillage; and
(d) separating the whole stillage into thin stillage and wet cake, whereby more liquid phase is transferred to the thin stillage,

wherein the cellulosic material is corn and the fermentation product is ethanol.

2. The method of claim 1, wherein the fermenting organism is a bacteria, yeast or fungi.

**Patentansprüche**

1. Verfahren zum Entwässern von Rohschlempe in einem Fermentationsverfahren, umfassend:

(a) Verzuckern eines Zellulosematerials mit einer Enzymzusammensetzung;
(b) Fermentieren des verzuckerten Zellulosematerials mit einem fermentierenden Organismus, um ein Fermentationsprodukt herzustellen, wobei das Fermentationsmedium eine Xylanase und eine Pektinase umfasst;
(c) Destillieren des Fermentationsproduktes zur Bildung der Rohschlempe; und
(d) Trennen der Rohschlempe in Dünnschlempe und Nasskuchen, wobei mehr flüssige Phase auf die Dünnschlempe übertragen wird,

wobei das Zellulosematerial Mais und das Fermentationsprodukt Ethanol ist.

2. Verfahren nach Anspruch 1, wobei der fermentierende Organismus ein Bakterium, eine Hefe oder ein Pilz ist.

**Revendications**

1. Procédé de déshydratation d'une vinasse entière dans un processus de fermentation, comprenant:

[a] la saccharification d'une matière cellulosique avec une composition d'enzyme ;
[b] la fermentation de la matière cellulosique saccharifiée avec un organisme de fermentation pour produire un produit de fermentation, dans lequel le milieu de fermentation comprend une xylanase et une pectinase ;
[c] la distillation du produit de fermentation pour former la vinasse entière ; et
[d] la séparation de la vinasse entière en vinasse fine et gâteau humide, moyennant quoi plus de phase liquide est transférée vers la vinasse fine,

dans lequel la matière cellulosique est du maïs et le produit de fermentation est de l'éthanol.

2. Procédé selon la revendication 1, dans lequel l'organisme de fermentation est une bactérie, une levure ou un champignon.

FIGURE 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012084225 A
- WO 2007056321 A
- WO 2011126897 A
- US 20050079270 A1
- WO 9421785 A
- WO 2006078256 A
- WO 2009079210 A
- US 5306633 A
- US 6433146 B
- US 7601858 B
- US 7608729 B
- US 20100058649 A